# EUROPEAN PATENT SPECIFICATION

(11) **EP 0 510 565 B1**
(45) Date of publication and mention of the grant of the patent: **25.06.1997**
(21) Application number: 92106754.2
(22) Date of filing: 21.04.1992
(51) Int. Cl.: C07H 15/04, C07H 15/08, C07C 309/17, C11D 1/12

(54) **Surface-active agents derived from sulfosuccinic esters**
Oberflächenaktive, von Sulfobernsteinsäureestern abgeleitete Verbindungen
Agents tensio-actifs dérivés d'esters d'acide sulfosuccinique

(30) Priority: 24.04.1991 IT MI911134
(43) Date of publication of application: 28.10.1992
(73) Proprietor: AUSCHEM S.P.A. in fallimento, 24100 Bergamo (IT)
(72) Inventor: Garlisi, Salvatore, I-13100 Vercelli (IT); Fornara, Dario, I-28100 Novara (IT); Bernardi, Paolo, I-20090 Vimodrone (MI) (IT)
(74) Representative: Giambrocono, Alfonso, Dr. Ing.

(56) References cited:
- EP-A- 0 454 321
- DE-A- 2 148 279
- DE-A- 2 304 057

## Description

### Description for the following Contracting States : BE, DK, GR, IT, LU, SE

The present invention relates to surface-active agents derived from sulfosuccinic di-esters and semiesters.

Sulfosuccinic surface-active agents derived from fatty alcohols possibly ethoxylated are already known. These surface-active agents have excellent skin tolerability, biodegradability, non-toxicity together with a fair foaming, emulsifying and wetting power.

They are consequently suitable for use in a wide field of applications, such as detergents in general, the food industry, textile industry, emulsion polymerization , etc.

The non-ethoxylated kinds however, are not soluble in water. The ethoxylated kinds must have an ethoxylation degree of at least 3 to 4 to show an acceptable solubility for detergent uses.

Ethoxylation, on the other hand, involves the use of ethylene oxide, which is dangerous for the operators, and also gives rise to the formation of reaction by-products which could be harmful even in small quantities, especially for the skin.

Surface-active agents have been found composed of sulfosuccinic diesters and semiesters which have excellent solubility in water even without oxyethylene units in their structure.

The present invention therefore relates to sulfosuccinic esters having the formula: 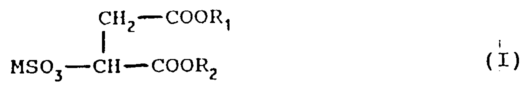 wherein:
M is Na, K, Mg NH₄ or their mixtures;
R₁ represents the group: 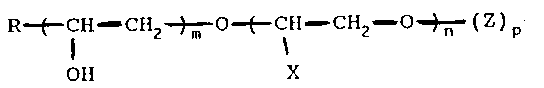
wherein:
R is an alkyl containing from 8 to 20 carbon atoms,
X is H or CH₃,
Z represents the residue of a hexose, pentose or sorbitol, with m which can be 0 to 1, n which can vary from 0 to 20, and
p which can vary from 1 to 10,
R₂ is M or R,
on the condition that when Z is the sorbitol residue, m=1, n=0 and p=1.

The cation M is preferably selected from sodium and the ammonium group.

The radical R is preferably selected from linear or branched alkyls containing from 8 to 14 carbon atoms. In particular, when m=1, the radical R is preferably an alkyl containing 8 carbon atoms.

A preferred group of compounds is that where in the R₁ group, Z represents the residue of glucose, m and n are equal to 0 and p is between 1 and 5.

Another group of preferred compounds is that wherein the R₁ group represents the residue of hydroxydecylsorbitol.

Sulfosuccinic esters having formula (I) are prepared by a process including the sulfonation of the respective maleic di-esters and semiesters having the formula: 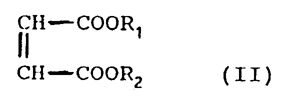 with the required sulfite or bisulfite of the cation M, wherein R₁, R₂ and M have the meaning specified above.

The reaction can be represented by the following equation:

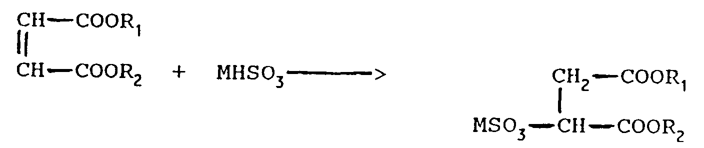

The maleic diesters and semiesters having formula (I) are well-known products and can be prepared by reacting maleic acid with alcohols having formulae R₁OH and R₂OH, wherein R₁ and R₂ have the meaning specified above.

The alcohols having formula R₁OH can be prepared by reacting a R₃OH alcohol with a hexose or pentose, wherein R₃ has the same meaning as R₁ except that p is equal to 0.

In the case of sorbitol, the alcohol R₁OH is prepared starting from alpha-epoxide in accordance with the following equation: 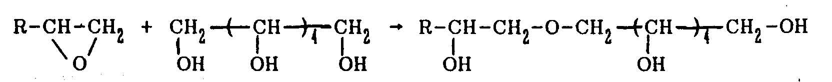 where R has the above-defined meaning.

In accordance with a specific aspect of the present invention, the alcohols having formula R₁OH can be mixed with even high quantities (up to 50% by weight and more) of unreacted alcohols having the formula R₃OH, preferably from 5 to 90%, more preferably from 70 to 80% when R₃ is an alkyl having a long chain; and from 10 to 20% when the alkyl has a short chain; from 20 to 70% when the chain of the alkyl has about intermediate values of the preferred R₁.

Alcohols having formula R₃OH, by reaction with maleic acid and subsequent sulfonation, produce sulfosuccinates having the formula:

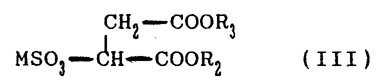

Both the esters corresponding to formula (I) of the present invention, and those correpsonding to formula (III), are very effective surface-active agents which, even if used in quite low percentages, allow a considerable decrease in surface tension and can therefore be used as emulsifiers, dispersers and detergents in general.

The solubility in water of the esters of the present invention is high, even in non-ethoxylated compounds, and is however higher with an equal degree of ethoxylation to the known sulfosuccinates, as is the surface tension, the foaming and imbibing power.

Besides their excellent detergent properties, they have no toxic or irritating effect on the skin or eyes and there is no acute toxicity if swallowed orally. In addition, they are highly biodegradable.

The sulfosuccinates of the present invention have proved to be compatible with most of the known anionic, amphoteric and non-ionic surface-active agents, and can consequently be formulated with these.

The esters of the present invention, because of their combination of characteristics, have proved to be very flexible in the various applications of surface-active agents.

Owing to their high detergent power together with a lack of toxic effects on the skin, hair and eyes, they are particularly suitable for cosmetic applications such as the preparation of liquid and cream detergents for the skin, shampoo and bath foam.

The following examples provide a better illustration of the present invention but do not limit it in any way.

### EXAMPLE 1

### Preparation of a sulfosuccinic semiester with a monoalkylpolyglucoside.

916 g (1 mole) of a maleic semiester of mixtures of polyglucosidated decyl and dodecyl alcohols, wherein the polyglucose has a polymerization degree of 3 to 4, 138.6 g (1.1 mole) of sodium sulfite and 158.9 g of distilled water are charged into a reactor equipped with a heating system, stirrer, thermometer and reagent inlet system.

The temperature is brought to 100°C under stirring and the reaction mixture is kept at this temperature for three hours.

The mixture is cooled to room temperature and is discharged from the reactor.

2636.5 g of a yellowish-white pasty product is obtained, containing 60% of water, having a 5% solution of clear active substance with a pH equal to 6.8 and organic SO₃ content of 2.9%.

### EXAMPLE 2

### Preparation of a sulfosuccinic semiester with a monoalkylpolyglucoside.

Using the same procedure as Example 1, 646 g (1 mole) of a maleic semiester prepared from a polyglucose with a polymerization degree of 3 and 4 etherified with a mixture of dodecyl alcohol and tetradecyl alcohol, are reacted with 138.6 g (1.1 moles) of sodium sulfite and 1626.9 g of distilled water.

2711.5 g of a white pasty solid product are obtained, containing 60% of water, having a 5% solution of active substance with a pH equal to 6.9 and organic SO₃ content of 3.0%.

### EXAMPLE 3

### Preparation of a sulfosuccinic semiester with a hydroxyalkylsorbitol.

Using the same procedure as Example 1, 436 g (1.0 moles) of a maleic semiester prepared from hydroxydecylsorbitol, are reacted with 138.6 g (1.1 moles) of sodium sulfite and 861.9 g of distilled water.

1436.5 g of a white, pasty fluid product are obtained, containing 60% of water, having a 5% solution of active substance with a pH equal to 6.8 and organic SO₃ content of 5.5%.

### EXAMPLE 4

### Preparation of a sulfosuccinic semiester with a monoalkylpolyglucoside.

500 g of a maleic ester, at 50% in water, prepared from a polyglucoside having a polymerization degree of 2.6 etherified with a mixture of decyl alcohol and dodecyl alcohol containing 29% by weight of the above mixture of alcohols in free form, 87.8 g of sodium sulfite and 256.7 g of distilled water are loaded into a reactor equipped with a heating system, stirrer, thermometer and reagent inlet system.

The temperature is brought to 100°C under stirring and the reaction mixture is kept at this temperature for three hours. The mass is already very clear after thirty minutes of reaction.

The mixture is cooled to room temperature and is discharged from the reactor.

844.5 g of a yellow clear liquid product are obtained, containing 60% of water, having a 5% solution of clear active substance with a pH equal to 7.0 and organic SO₃ content of 6.3%.

### EXAMPLE 5

### Preparation of a sulfosuccinic semiester with a monoalkylpolyglucoside.

Using the same procedure as Example 4, 450.0 g of a maleic ester, at 50% in water, prepared from a polyglucose having a polymerization degree of 3.9 etherified with a mixture of dodecyl and tetradecyl alcohols, containing 38% by weight of the above mixture of alcohols in free form, are reacted with 87.1 g of sodium sulfite and 243.1 g of distilled water.

780.2 g of a white, pasty solid product are obtained, containing 60% of water, having a 5% solution of clear active substance with a pH equal to 7.1 and organic SO₃ content of 5.9%.

### APPLICATIVE TESTS

The following tests were carried out on the products obtained in Examples 1-5 and on the sulfosuccinic semiester of C₁₂-C₁₄ fatty alcohols, sodium salt, (Example A as a comparison):
- Surface tension
   The surface tension of solutions at 0.1% of active substance was measured at 20°C using the du-Nouy method. The results, measured in N/m (dine/cm) at a concentration of 1 g/l, are shown in the Table.
- Foaming power
   The capacity of producing foam was evaluated using the Ross-Miles method at 25°C. Measurements were made in distilled water at concentrations of 0.5 g/1 after 0 minutes (I) and after 5 minutes (II), and at concentrations of 1 g/1 after 0 minutes (III) and after 5 minutes (IV). The same measurements were repeated in hard water 5 mg/l Ca (30°F) (V-VIII). The results are shown in the Table.
- Imbibing power
   The imbibing power was evaluated by measuring the time taken by a special cotton planchet, completely immersed in a solution at 0.2% of active substance of the product being tested, to fall to the bottom of the container. The results are shown in the Table.
- Solubility
   5% aqueous solutions of the compounds obtained in Examples 1, 2 and 3 are very clear, whereas in the case of comparative Example A, the appearance is decidedly cloudy.

**TABLE**

| Example | Surface tension | Foaming power | | | | | | | | Imbibing power |
|---|---|---|---|---|---|---|---|---|---|---|
| | | I | II | III | IV | V | VI | VII | VIII | |
| 1 | 0.0275 | 40 | 40 | 55 | 55 | 40 | 30 | 45 | 40 | 100 |
| 2 | 0.0276 | 60 | 55 | 100 | 95 | 45 | 40 | 100 | 95 | 60 |
| 3 | 0.0265 | 65 | 60 | 110 | 100 | 25 | 20 | 45 | 35 | 98 |
| 4 | 0.0290 | 40 | 30 | 55 | 50 | 45 | 40 | 145 | 140 | 96 |
| 5 | 0.0305 | 60 | 55 | 90 | 85 | 110 | 105 | 130 | 125 | 55 |
| A | 0.0341 | 20 | 15 | 20 | 15 | 15 | 15 | 15 | 15 | 155 |

### Description for the following Contracting States : AT, CH, DE, ES, FR, GB, LI, NL

The present invention relates to surface-active agents derived from sulfosuccinic di-esters and semiesters.

Sulfosuccinic surface-active agents derived from fatty alcohols possibly ethoxylated are already known. These surface-active agents have excellent skin tolerability, biodegradability, non-toxicity together with a fair foaming, emulsifying and wetting power.

They are consequently suitable for use in a wide field of applications, such as detergents in general, the food industry, textile industry, emulsion polymerization , etc.

The non-ethoxylated kinds however, are not soluble in water. The ethoxylated kinds must have an ethoxylation degree of at least 3 to 4 to show an acceptable solubility for detergent uses.

Ethoxylation, on the other hand, involves the use of ethylene oxide, which is dangerous for the operators, and also gives rise to the formation of reaction by-products which could be harmful even in small quantities, especially for the skin.

EP-A-0 454 321, unpublished at the filing date of the present application, discloses glycoside esters of solfosuccinic acid.

Surface-active agents have been found composed of sulfosuccinic diesters and semiesters which have excellent solubility in water even without oxyethylene units in their structure.

The present invention therefore relates to sulfosuccinic esters having the formula: 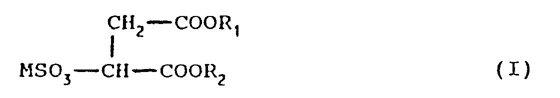 wherein:
M is Na, K, Mg NH₄ or their mixtures;
R₁ represents the group: 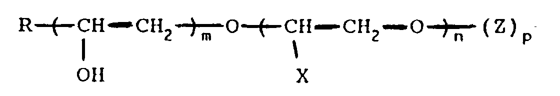
wherein:
R is an alkyl containing from 8 to 20 carbon atoms,
X is H or CH₃,
Z represents the residue of a hexose, pentose or sorbitol, with m which can be 0 to 1, n which can vary from 0 to 20, and
p which can vary from 1 to 10,
R₂ is M or R,
on the condition that when Z is the sorbitol residue, m=1, n=0 and p=1.

The cation M is preferably selected from sodium and the ammonium group.

The radical R is preferably selected from linear or branched alkyls containing from 8 to 14 carbon atoms. In particular, when m=1, the radical R is preferably an alkyl containing 8 carbon atoms.

A preferred group of compounds is that where in the R₁ group, Z represents the residue of glucose, m and n are equal to 0 and p is between 1 and 5.

Another group of preferred compounds is that wherein the R₁ group represents the residue of hydroxydecylsorbitol.

Sulfosuccinic esters having formula (I) are prepared by a process including the sulfonation of the respective maleic di-esters and semiesters having the formula: 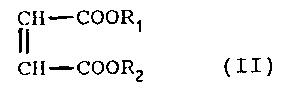 with the required sulfite or bisulfite of the cation M, wherein R₁, R₂ and M have the meaning specified above.

The reaction can be represented by the following equation:

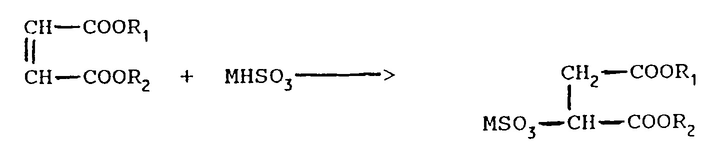

The maleic diesters and semiesters having formula (I) are well-known products and can be prepared by reacting maleic acid with alcohols having formulae R₁OH and R₂OH, wherein R₁ and R₂ have the meaning specified above.

The alcohols having formula R₁OH can be prepared by reacting a R₃OH alcohol with a hexose or pentose, wherein R₃ has the same meaning as R₁ except that p is equal to 0.

In the case of sorbitol, the alcohol R₁OH is prepared starting from alpha-epoxide in accordance with the following equation: 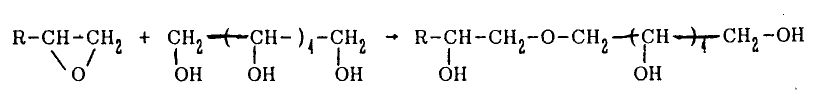 where R has the above-defined meaning.

In accordance with a specific aspect of the present invention, the alcohols having formula R₁OH can be mixed with even high quantities (up to 50% by weight and more) of unreacted alcohols having the formula R₃OH, preferably from 5 to 90%, more preferably from 70 to 80% when R₃ is an alkyl having a long chain; and from 10 to 20% when the alkyl has a short chain; from 20 to 70% when the chain of the alkyl has about intermediate values of the preferred R₁.

Alcohols having formula R₃OH, by reaction with maleic acid and subsequent sulfonation, produce sulfosuccinates having the formula:

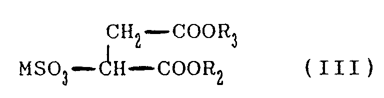

Both the esters corresponding to formula (I) of the present invention, and those correpsonding to formula (III), are very effective surface-active agents which, even if used in quite low percentages, allow a considerable decrease in surface tension and can therefore be used as emulsifiers, dispersers and detergents in general.

The solubility in water of the esters of the present invention is high, even in non-ethoxylated compounds, and is however higher with an equal degree of ethoxylation to the known sulfosuccinates, as is the surface tension, the foaming and imbibing power.

Besides their excellent detergent properties, they have no toxic or irritating effect on the skin or eyes and there is no acute toxicity if swallowed orally. In addition, they are highly biodegradable.

The sulfosuccinates of the present invention have proved to be compatible with most of the known anionic, amphoteric and non-ionic surface-active agents, and can consequently be formulated with these.

The esters of the present invention, because of their combination of characteristics, have proved to be very flexible in the various applications of surface-active agents.

Owing to their high detergent power together with a lack of toxic effects on the skin, hair and eyes, they are particularly suitable for cosmetic applications such as the preparation of liquid and cream detergents for the skin, shampoo and bath foam.

The following examples provide a better illustration of the present invention but do not limit it in any way.

### EXAMPLE

### Preparation of a sulfosuccinic semiester with a hydroxyalkylsorbitol.

Using the same procedure as Example 1, 436 g (1.0 moles) of a maleic semiester prepared from hydroxydecylsorbitol, are reacted with 138.6 g (1.1 moles) of sodium sulfite and 861.9 g of distilled water.

1436.5 g of a white, pasty fluid product are obtained, containing 60% of water, having a 5% solution of active substance with a pH equal to 6.8 and organic SO₃ content of 5.5%.

### APPLICATIVE TESTS

The following tests were carried out on the products obtained in the Example and on the sulfosuccinic semiester of C₁₂-C₁₄ fatty alcohols, sodium salt, (Example A as a comparison):
- Surface tension
   The surface tension of solutions at 0.1% of active substance was measured at 20°C using the du-Nouy method. The results, measured in N/m (dine/cm) at a concentration of 1 g/l, are shown in the Table.
- Foaming power
   The capacity of producing foam was evaluated using the Ross-Miles method at 25°C. Measurements were made in distilled water at concentrations of 0.5 g/1 after 0 minutes (I) and after 5 minutes (II), and at concentrations of 1 g/1 after 0 minutes (III) and after 5 minutes (IV). The same measurements were repeated in hard water 5 mg/l Ca (30°F) (V-VIII). The results are shown in the Table.
- Imbibing power
   The imbibing power was evaluated by measuring the time taken by a special cotton planchet, completely immersed in a solution at 0.2% of active substance of the product being tested, to fall to the bottom of the container. The results are shown in the Table.
- Solubility
   5% aqueous solution of the compound obtained in Example is very clear, whereas in the case of comparative Example A, the appearance is decidedly cloudy.

**TABLE**

| Example | Surface tension | Foaming power | | | | | | | | Imbibing power |
|---|---|---|---|---|---|---|---|---|---|---|
| | | I | II | III | IV | V | VI | VII | VIII | |
| 3 | 0.0265 | 65 | 60 | 110 | 100 | 25 | 20 | 45 | 35 | 98 |
| A | 0.0341 | 20 | 15 | 20 | 15 | 15 | 15 | 15 | 15 | 155 |

## Claims (Claims for the following Contracting State(s): BE, DK, IT, LU, SE)

1. Sulfosuccinic esters having the formula: 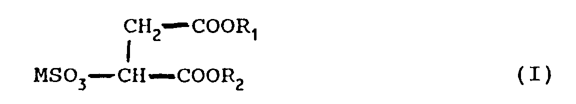 wherein:
M is Na, K, Mg, NH₄ or their mixtures;
R₁ represents the group: 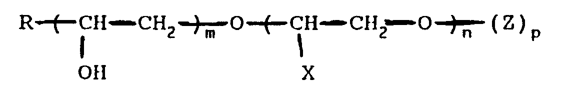
wherein:
R is an alkyl containing from 8 to 20 carbon atoms,
X is H or CH₃,
Z represents the residue of a hexose, pentose or sorbitol,
with m which can be 0 or 1, n which can vary from 0 to 20, and p which can vary from 1 to 10,
R₂ is M or R,
on the condition that when Z is the residue of sorbitol, m=1, n=0 and p=1.

2. Esters in accordance with Claim 1, wherein Z represents the residue of glucose, m and n are equal to 0 and p is between 1 and 5.

3. Esters in accordance with Claim 1, mixed with sulfosuccinic esters having the formula: 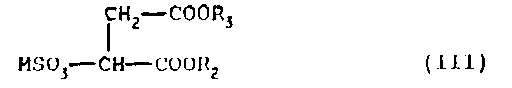 where R₃ has the same meaning as R₁ except that p is equal to 0.

4. Sulfosuccinic esters having the formula: 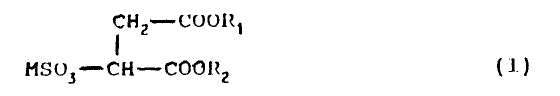 wherein:
M is Na, K, Mg, NH₄ or their mixtures;
R₁ represents the group: 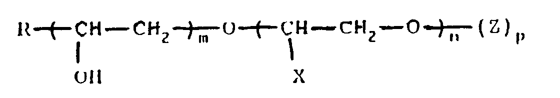
wherein:
R is an alkyl containing from 8 to 20 carbon atoms,
X is H or CH₃,
Z represents the residue of a hexose, pentose or sorbitol,
with m which can be 0 or 1, n which can vary from 0 to 20, and p which can vary from 1 to 10,
R₂ is H or R,
on the condition that when Z is the residue of sorbitol, m=1, n=0 and p=1; and the condition that when Z is an hexose or a pentose and R₂ is M then m is 1, or n is 1-20 and X is CH₃.

5. Cosmetic and/or detergent compositions containing one or more sulfosuccinic esters in accordance with Claim 1 to 4.

6. Detergent compositions in accordance with Claim 5, wherein the sulfosuccinic esters are mixed with other surface-active agents.

## Claims (Claims for the following Contracting State(s): AT, CH, DE, FR, GB, LI, NL)

1. Sulfosuccinic esters having the formula: 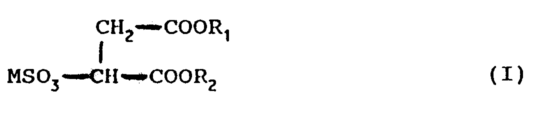 wherein:
M is Na, K, Mg, NH₄ or their mixtures;
R₁ represents the group: 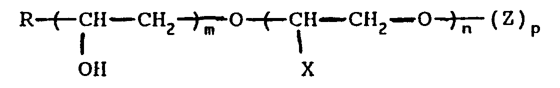
wherein:
R is an alkyl containing from 8 to 20 carbon atoms,
X is H or CH₃,
Z represents the residue of a hexose, pentose or sorbitol,
with m which can be 0 or 1, n which can vary from 0 to 20, and p which can vary from 1 to 10,
R₂ is M or R,
on the condition that when Z is the residue of sorbitol, m=1, n=0 and p=1 and that when Z is a hexose or a pentose and R₂ is M, m is 1.

2. Esters in accordance with Claim 1, except that, when Z is a hexose or a pentose, m is both 0 and 1, mixed with sulfosuccinic esters having the formula: 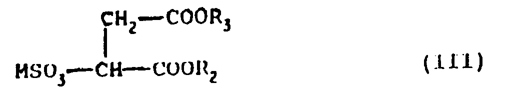 where R₃ has the same meaning as R₁ except that p is equal to 0.

3. Sulfosuccinic esters having the formula: 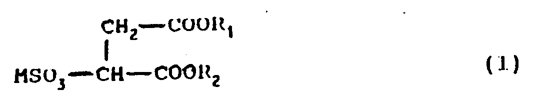 wherein:
M is Na, K, Mg, NH₄ or their mixtures;
R₁ represents the group: 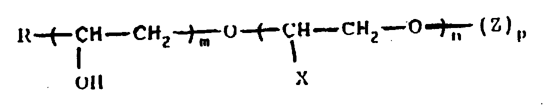
wherein:
R is an alkyl containing from 8 to 20 carbon atoms,
X is H or CH₃,
Z represents the residue of a hexose, pentose or sorbitol,
with m which can be 0 or 1, n which can vary from 0 to 20, and p which can vary from 1 to 10,
R₂ is H or R,
on the condition that when Z is the residue of sorbitol, m=1, n=0 and p=1; and the condition that when Z is an hexose or a pentose and R₂ is M then m is 1, or n is 1-20 and X is CH₃.

4. Cosmetic and/or detergent compositions containing one or more sulfosuccinic esters in accordance with Claim 1 to 3.

5. Detergent compositions in accordance with Claim 4, wherein the sulfosuccinic esters are mixed with other surface-active agents.

## Claims (Claims for the following Contracting State(s): ES)

1. A process for the preparation of sulfosuccinic esters having the formula: 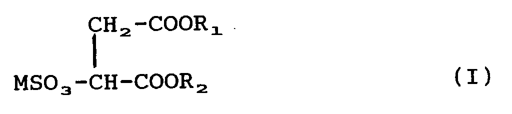 wherein:
M is Na, K, Mg, NH₄ or their mixtures;
R₁ represents the group: 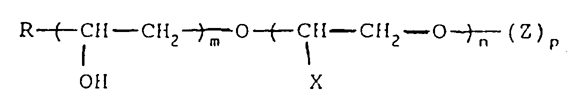
wherein:
R is an alkyl containing from 8 to 20 carbon atoms,
X is H or CH₃,
Z represents the residue of a hexose, pentose or sorbitol,
with m which can be 0 or 1, n which can vary from 0 to 20, and p which can vary from 1 to 10,
R₂ is M or R,
on the condition that when Z is the residue of sorbitol, m=1, n=0 and p=1 and that when Z is a hexose or a pentose and R₂ is M, m is 1,
including the sulfonation of the respective maleic di-esters and semiesters having the formula: 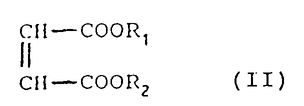 with the required sulfite or bisulfite of the cation M, wherein R₁, R₂ and M have the meaning specified above.

2. A process for the preparation of esters in accordance with claim 1, except that, when Z is a hexose or a pentose m is both 0 and 1, which further comprises mixing the resulting esters with sulfosuccinic esters having the formula: 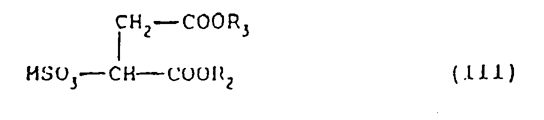 where R₃ has the same meaning as R₁ except that p is equal to 0.

3. A process for the preparation of sulfosuccinic esters having the formula: 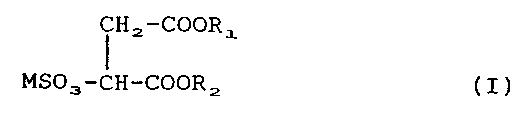 wherein is Na, K, Mg, NH₄ or their mixtures;
R₁ represents the group: 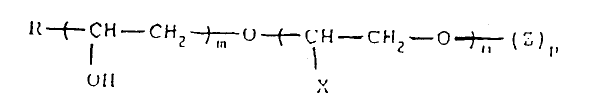 wherein:
R is an alkyl containing from 8 to 20 carbon atoms,
X is H or CH₃,
Z represents the residue of a hexose, pentose or sorbitol,
with m which can be 0 or 1, n which can vary from 0 to 20, and p which can vary from 1 to 10,
R₂ is M or R,
on the condition that when Z is the residue of sorbitol, m=1, n=0 and p=1; and the condition that when Z is an hexose or a pentose and R₂ is M then m is 1, or n is 1-20 and X is CH₃,
including the sulfonation of the respective maleic di-esters and semiesters having the formula: 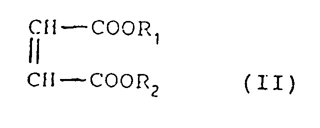 with the required sulfite or bisulfite of the cation M, wherein R₁, R₂ and M have the meaning specified above.

4. A process for the preparation of cosmetic and/or detergent compositions containing one or more sulfosuccinic esters in accordance with claim 1 to 3 which comprises mixing at least one of said esters with at least one suitable vehicle.

5. A process for the preparation of detergent compositions in accordance with claim 4, wherein the sulfosuccinic esters are mixed with other surface-active agents.

## Claims (Claims for the following Contracting State(s): GR)

1. A process for the preparation of sulfosuccinic esters having the formula: 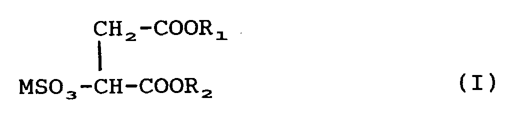 wherein:
M is Na, K, Mg, NH₄ or their mixtures;
R₁ represents the group: 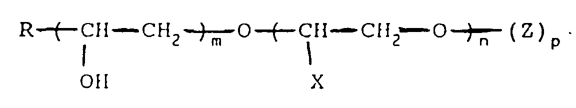
wherein:
R is an alkyl containing from 8 to 20 carbon atoms,
X is H or CH₃,
Z represents the residue of a hexose, pentose or sorbitol,
with m which can be 0 or 1, n which can vary from 0 to 20, and p which can vary from 1 to 10,
R₂ is M or R,
on the condition that when Z is the residue of sorbitol, m=1, n=0 and p=1,
including the sulfonation of the respective maleic di-esters and semiesters having the formula: 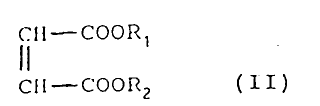 with the required sulfite or bisulfite of the cation M, wherein R₁, R₂ and M have the meaning specified above.

2. A process for the preparation of esters in accordance with claim 1, wherein Z represents the residue of glucose, m and n are equal to O and p is between 1 and 5.

3. A process for the preparation of esters in accordance with claim 1, which further comprises mixing the resulting esters with sulfosuccinic esters having the formula: 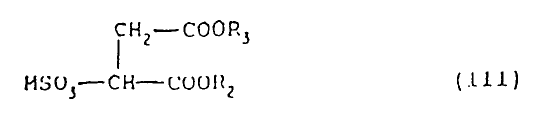 where R₃ has the same meaning as R₁ except that p is equal to 0.

4. A process for the preparation of esters having the formula: 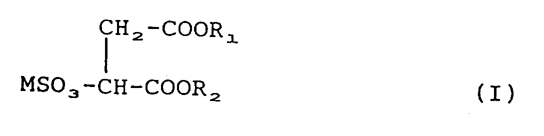 wherein is Na, K, Mg, NH₄ or their mixtures;
R₁ represents the group: 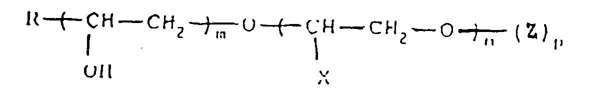 wherein:
R is an alkyl containing from 8 to 20 carbon atoms,
X is H or CH₃,
Z represents the residue of a hexose, pentose or sorbitol,
with m which can be 0 or 1, n which can vary from 0 to 20, and p which can vary from 1 to 10,
R₂ is M or R,
on the condition that when Z is the residue of sorbitol, m=1, n=0 and p=1; and the condition that when Z is an hexose or a pentose and R₂ is M then m is 1, or n is 1-20 and X is CH₃,
including the sulfonation of the respective maleic di-esters and semiesters having the formula: 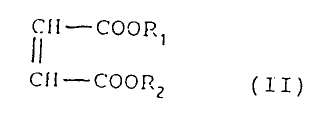 with the required sulfite or bisulfite of the cation M, wherein R₁, R₂ and M have the meaning specified above.

5. A process for the preparation of cosmetic and/or detergent compositions containing one or more sulfosuccinic esters in accordance with claim 1 to 4 which comprises mixing at least one of said esters with at least one suitable vehicle.

6. A process for the preparation of detergent compositions in accordance with claim 5, wherein the sulfosuccinic esters are mixed with other surface-active agents.

## Patentansprüche (Patentansprüche für folgende(n) Vertragsstaat(en): BE, DK, IT, LU, SE)

1. Sulfobernsteinsäureester der Formel: 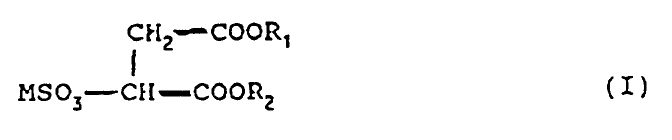 worin
M für Na, K, Mg, NH₄ oder ihre Gemische steht;
R₁ die Gruppe: 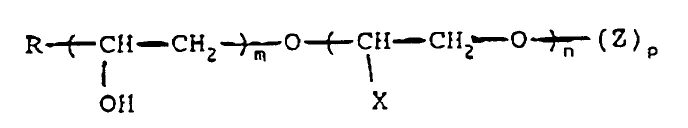
bedeutet, worin
R für Alkyl, enthaltend 8 bis 20 Kohlenstoffatome, steht;
X für H oder CH₃ steht;
Z den Rest einer Hexose, Pentose oder von Sorbit bedeutet;
wobei m den Wert 0 oder 1 haben kann, n von 0 bis 20 variieren kann und p von 1 bis 10 variieren kann;
R₂ für M oder R steht,
mit der Maßgabe, daß, wenn Z den Rest von Sorbit bedeutet, m=1, n=0 und p=1.

2. Ester nach Anspruch 1, dadurch **gekennzeichnet,** daß Z für den Rest von Glucose steht, m und n den Wert 0 haben und p zwischen 1 und 5 liegt.

3. Ester nach Anspruch 1 im Gemisch mit Sulfobernsteinsäureestern der Formel: 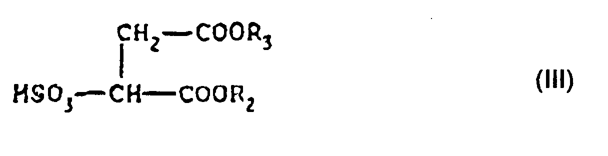 worin
R₃ die gleiche Bedeutung wie R₁ hat, mit der Ausnahme, daß p den Wert 0 hat.

4. Sulfobernsteinsäureester mit der Formel: 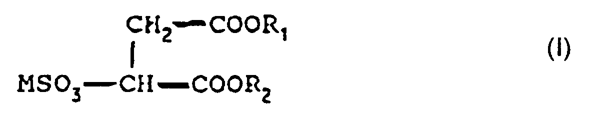 worin
M für Na, K, Mg, NH₄ oder ihre Gemische steht;
R₁ die Gruppe: 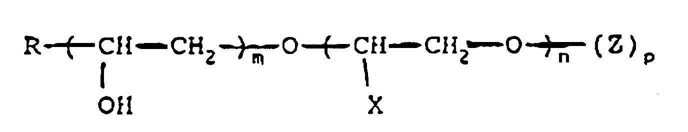
bedeutet, worin
R für Alkyl, enthaltend 8 bis 20 Kohlenstoffatome, steht;
X für H oder CH₃ steht;
Z den Rest einer Hexose, Pentose oder von Sorbit bedeutet;
wobei m den Wert 0 oder 1 haben kann, n von 0 bis 20 variieren kann und p von 1 bis 10 variieren kann;
R₂ für M oder R steht,
mit der Maßgabe, daß, wenn Z den Rest von Sorbit bedeutet, m=1, n=0 und p=1; und mit der Maßgabe, daß, wenn Z eine Hexose oder eine Pentose ist und R₂ für M steht, dann m den Wert 1 hat oder n 1 bis 20 ist und X für CH₃ steht.

5. Kosmetische und/oder Detergenszubereitungen, enthaltend ein oder mehrere Sulfobernsteinsäureester nach den Ansprüchen 1 bis 4.

6. Detergenszubereitungen nach Anspruch 5, dadurch **gekennzeichnet,** daß die Sulfobernsteinsäureester im Gemisch mit anderen oberflächenaktiven Mitteln vorliegen.

## Patentansprüche (Patentansprüche für folgende(n) Vertragsstaat(en): AT, CH, DE, FR, GB, LI, NL)

1. Sulfobernsteinsäureester der Formel: 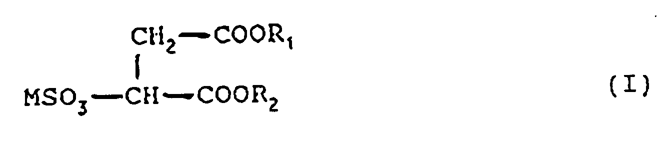 worin
M für Na, K, Mg, NH₄ oder ihre Gemische steht;
R₁ die Gruppe: 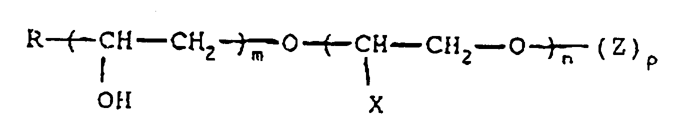
bedeutet, worin
R für Alkyl, enthaltend 8 bis 20 Kohlenstoffatome, steht;
X für H oder CH₃ steht;
Z den Rest einer Hexose, Pentose oder von Sorbit bedeutet;
wobei m den Wert 0 oder 1 haben kann, n von 0 bis 20 variieren kann und p von 1 bis 10 variieren kann;
R₂ für M oder R steht,
mit der Maßgabe, daß, wenn Z den Rest von Sorbit bedeutet, m=1, n=0 und p=1, und daß, wenn Z für eine Hexose oder eine Pentose steht und R₂ für M steht, m den Wert 1 hat.

2. Ester nach Anspruch 1, mit der Ausnahme, daß, wenn Z eine Hexose oder eine Pentose ist, m sowohl den Wert 0 als auch 1 hat, im Gemisch mit Sulfobernteinsäureestern mit der Formel: 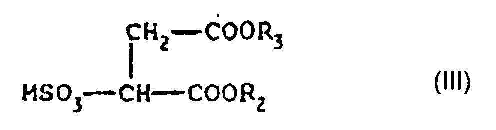 worin R₃ die gleiche Bedeutung wie R₁ hat, ausgenommen, daß p den Wert 0 hat.

3. Sulfobernsteinsäureester mit der Formel: 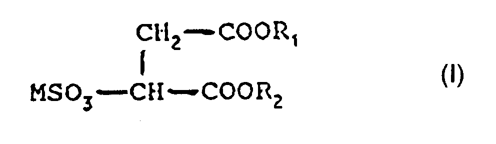 worin
M für Na, K, Mg, NH₄ oder ihre Gemische steht;
R₁ die Gruppe: 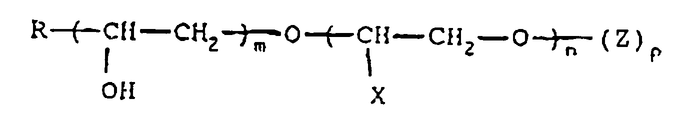
bedeutet, worin
R für Alkyl, enthaltend 8 bis 20 Kohlenstoffatome, steht;
X für H oder CH₃ steht;
Z den Rest einer Hexose, Pentose oder von Sorbit bedeutet;
wobei m den Wert 0 oder 1 haben kann, n von 0 bis 20 variieren kann und p von 1 bis 10 variieren kann;
R₂ für M oder R steht,
mit der Maßgabe, daß, wenn Z den Rest von Sorbit bedeutet, m=1, n=0 und p=1; und mit der Maßgabe, daß, wenn Z eine Hexose oder eine Pentose ist und R₂ für M steht, dann m den Wert 1 hat oder n 1 bis 20 ist und X für CH₃ steht.

4. Kosmetische und/oder Detergenszubereitungen, enthaltend ein oder mehrere Sulfobernsteinsäureester nach den Ansprüchen 1 bis 3.

5. Detergenszubereitungen nach Anspruch 4, dadurch **gekennzeichnet,** daß die Sulfobernsteinsäureester im Gemisch mit anderen oberflächenaktiven Mitteln vorliegen.

## Patentansprüche (Patentansprüche für folgende(n) Vertragsstaat(en): ES)

1. Verfahren zur Herstellung von Sulfobernsteinsäureestern der Formel: 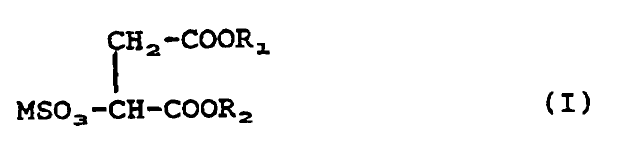 worin
M für Na, K, Mg, NH₄ oder ihre Gemische steht;
R₁ die Gruppe: 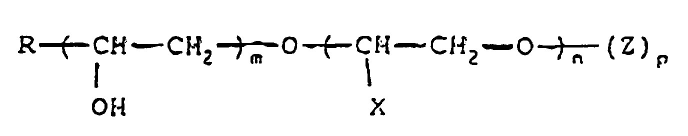
bedeutet, worin
R für Alkyl, enthaltend 8 bis 20 Kohlenstoffatome, steht;
X für H oder CH₃ steht;
Z den Rest einer Hexose, Pentose oder von Sorbit bedeutet;
wobei m den Wert 0 oder 1 haben kann, n von 0 bis 20 variieren kann und p von 1 bis 10 variieren kann;
R₂ für M oder R steht,
mit der Maßgabe, daß, wenn Z den Rest von Sorbit bedeutet, m=1, n=0 und p=1,und daß, wenn Z für eine Hexose oder eine Pentose steht und R₂ für M steht, m den Wert 1 hat,
das die Sulfonierung der jeweiligen Maleinsäurediester und -semiester mit der Formel: 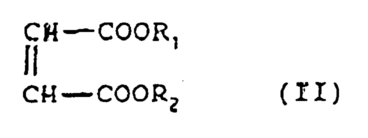 mit dem erforderlichen Sulfit oder Bisulfit des Kations M einschließt, wobei R₁, R₂ und M die oben angegebenen Bedeutungen haben.

2. Verfahren zur Herstellung von Estern nach Anspruch 1, ausgenommen, daß, wenn Z eine Hexose oder eine Pentose ist, m sowohl den Wert 0 als auch 1 hat, weiterhin umfassend die Vermischung der resultierenden Ester mit Sulfobernsteinsäureestern mit der Formel: 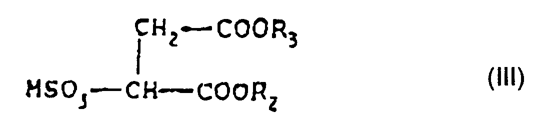 worin R₃ die gleiche Bedeutung wie R₁ hat, mit der Ausnahme, daß p den Wert 0 hat.

3. Verfahren zur Herstellung von Sulfobernsteinsäureestern mit der Formel: 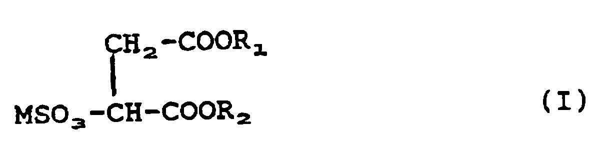 worin
M für Na, K, Mg, NH₄ oder ihre Gemische steht;
R₁ die Gruppe: 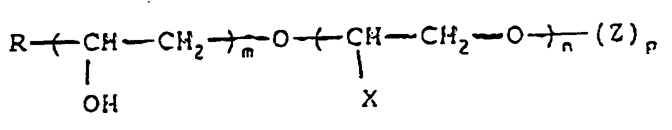
bedeutet, worin
R für Alkyl, enthaltend 8 bis 20 Kohlenstoffatome, steht;
X für H oder CH₃ steht;
Z den Rest einer Hexose, Pentose oder von Sorbit bedeutet;
wobei m den Wert 0 oder 1 haben kann, n von 0 bis 20 variieren kann und p von 1 bis 10 variieren kann;
R₂ für M oder R steht,
mit der Maßgabe, daß, wenn Z den Rest von Sorbit bedeutet, m=1, n=0 und p=1; und mit der Maßgabe, daß, wenn Z für eine Hexose oder eine Pentose steht und R₂ für M steht, dann m den Wert 1 hat oder n 1 bis 20 ist und X für CH₃ steht,
das die Sulfonierung der jeweiligen Maleinsäurediester und -semiester mit der Formel: 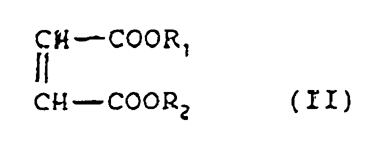 mit dem erforderlichen Sulfit oder Bisulfit des Kations M einschließt, wobei R₁, R₂ und M die oben angegebenen Bedeutungen haben.

4. Verfahren zur Herstellung von kosmetischen und/oder Detergenszubereitungen, die ein oder mehrere Sulfobernsteinsäureester nach den Ansprüchen 1 bis 3 enthalten, umfassend die Vermischung von mindestens einem der genannten Ester mit mindestens einem geeigneten Träger.

5. Verfahren zur Herstellung von Detergenszubereitungen nach Anspruch 4, dadurch **gekennzeichnet,** daß man die Sulfobernsteinsäureester mit anderen oberflächenaktiven Mitteln vermischt.

## Patentansprüche (Patentansprüche für folgende(n) Vertragsstaat(en): GR)

1. Verfahren zur Herstellung von Sulfobernsteinsäureestern der Formel: 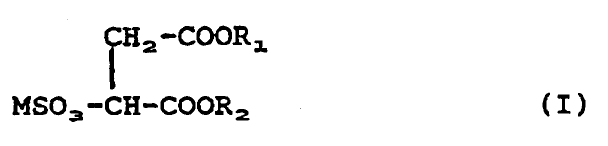 worin
M für Na, K, Mg, NH₄ oder ihre Gemische steht;
R₁ die Gruppe: 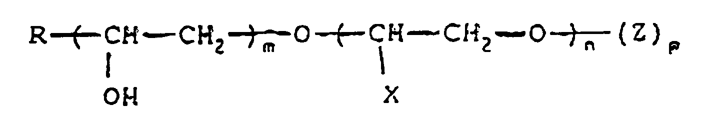
bedeutet, worin
R für Alkyl, enthaltend 8 bis 20 Kohlenstoffatome, steht;
X für H oder CH₃ steht;
Z den Rest einer Hexose, Pentose oder von Sorbit bedeutet;
wobei m den Wert 0 oder 1 haben kann, n von 0 bis 20 variieren kann und p von 1 bis 10 variieren kann;
R₂ für M oder R steht,
mit der Maßgabe, daß, wenn Z den Rest von Sorbit bedeutet, m=1, n=0 und p=1,
das die Sulfonierung derjeweiligen Maleinsäurediester und -semiester mit der Formel: 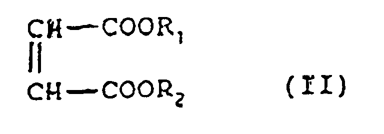 mit dem erforderlichen Sulfit oder Bisulfit des Kations M einschließt, wobei R₁, R₂ und M die oben angegebenen Bedeutungen haben.

2. Verfahren zur Herstellung von Estern nach Anspruch 1, dadurch **gekennzeichnet,** daß Z für den Rest von Glucose steht, m und n den Wert 0 haben und p zwischen 1 und 5 liegt.

3. Verfahren zur Herstellung von Estern nach Anspruch 1, weiterhin umfassend die Vermischung der resultierenden Ester mit Sulfobernsteinsäureestern mit der Formel: 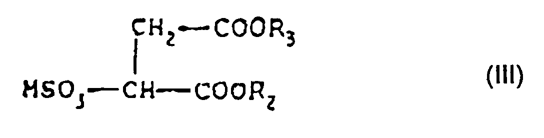 worin R₃ die gleiche Bedeutung wie R₁ hat, mit der Ausnahme, daß p den Wert 0 hat.

4. Verfahren zur Herstellung von Estern mit der Formel: 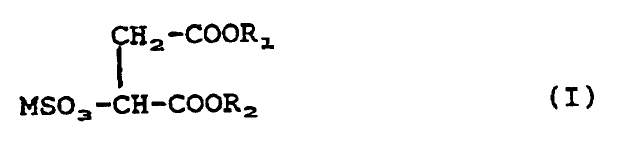 worin
M für Na, K, Mg, NH₄ oder ihre Gemische steht;
R₁ die Gruppe: 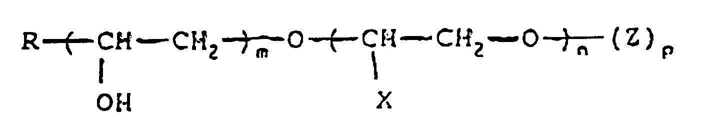
bedeutet, worin
R für Alkyl, enthaltend 8 bis 20 Kohlenstoffatome, steht;
X für H oder CH₃ steht;
Z den Rest einer Hexose, Pentose oder von Sorbit bedeutet;
wobei m den Wert 0 oder 1 haben kann, n von 0 bis 20 variieren kann und p von 1 bis 10 variieren kann;
R₂ für M oder R steht,
mit der Maßgabe, daß, wenn Z den Rest von Sorbit bedeutet, m=1, n=0 und p=1; und mit der Maßgabe, daß, wenn Z für eine Hexose oder eine Pentose steht und R₂ für M steht, dann m den Wert 1 hat, oder n 1 bis 20 ist und X für CH₃ steht,
das die Sulfonierung der jeweiligen Maleinsäurediester und -semiester mit der Formel: 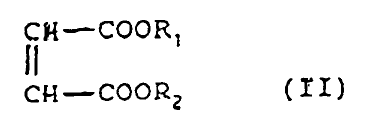 mit dem erforderlichen Sulfit oder Bisulfit des Kations M einschließt, wobei R₁, R₂ und M die oben angegebenen Bedeutungen haben.

5. Verfahren zur Herstellung von kosmetischen und/oder Detergenszubereitungen, die ein oder mehrere Sulfobernsteinsäureester nach den Ansprüchen 1 bis 4 enthalten, umfassend die Vermischung von mindestens einem der genannten Ester mit mindestens einem geeigneten Träger.

6. Verfahren zur Herstellung von Detergenszubereitungen nach Anspruch 5, dadurch **gekennzeichnet,** daß man die Sulfobernsteinsäureester mit anderen oberflächenaktiven Mitteln vermischt.

## Revendications (Revendications pour l'(les) Etat(s) contractant(s) suivant(s): BE, DK, IT, LU, SE)

1. Esters de l'acide sulfosuccinique ayant la formule : 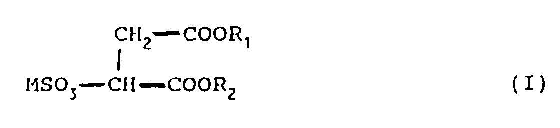 dans laquelle :
M est Na, K, Mg, NH₄ ou leurs mélanges ;
R₁ représente le groupe : 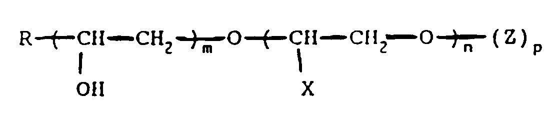
dans lequel :
R est un groupe alkyle ayant de 8 à 20 atomes de carbone,
X est H ou CH₃,
Z représente le résidu d'un hexose, d'un pentose ou du sorbitol,
m pouvant valoir 0 ou 1, n pouvant varier de 0 à 20 et p pouvant varier de 1 à 10,
R₂ est M ou R,
à la condition que, quand Z est le résidu du sorbitol, m = 1, n = 0 et p = 1.

2. Esters selon la revendication 1, dans lesquels Z représente le résidu du glucose, m et n valent chacun 0 et p est compris entre 1 et 5.

3. Esters selon la revendication 1, en mélange avec des esters de l'acide sulfosuccinique ayant la formule : 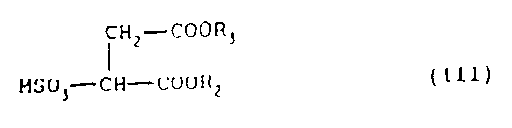 dans laquelle R₃ a les mêmes significations que R₁, sauf que p est égal à 0.

4. Esters de l'acide sulfosuccinique ayant la formule : 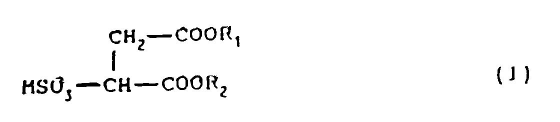 dans laquelle :
M est Na, K, Mg, NH₄ ou leurs mélanges ;
R₁ représente le groupe : 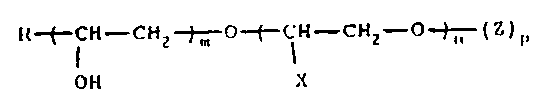
dans lequel :
R est un groupe alkyle ayant de 8 à 20 atomes de carbone,
X est H ou CH₃,
Z représente le résidu d'un hexose, d'un pentose ou du sorbitol,
m pouvant valoir 0 ou 1, n pouvant varier de 0 à 20 et p pouvant varier de 1 à 10,
R₂ est M ou R,
à la condition que, quand Z est le résidu du sorbitol, m = 1, n = 0 et p = 1 ; et à la condition que, quand Z est un hexose ou un pentose et R₂ est M, alors m vaut 1, ou n vaut de 1 à 20 et X est CH₃.

5. Compositions cosmétiques et/ou détergentes contenant un ou plusieurs esters de l'acide sulfosuccinique selon les revendications 1 à 4.

6. Compositions détergentes selon la revendication 5, dans lesquelles les esters de l'acide sulfosuccinique sont mélangés à d'autres agents tensioactifs.

## Revendications (Revendications pour l'(les) Etat(s) contractant(s) suivant(s): AT, CH, DE, FR, GB, LI, NL)

1. Esters de l'acide sulfosuccinique ayant la formule: 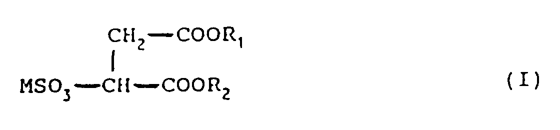 dans laquelle :
M est Na, K, Mg, NH₄ ou leurs mélanges ;
R₁ représente le groupe : 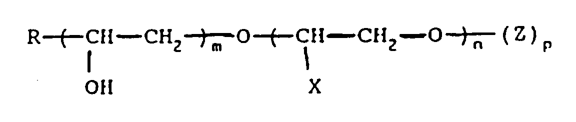
dans lequel :
R est un groupe alkyle ayant de 8 à 20 atomes de carbone,
X est H ou CH₃,
Z représente le résidu d'un hexose, d'un pentose ou du sorbitol,
m pouvant valoir 0 ou 1, n pouvant varier de 0 à 20 et p pouvant varier de 1 à 10,
R₂ est M ou R,
à la condition que, quand Z est le résidu de sorbitol, m = 1, n = 0 et p = 1, et que, quand Z est un hexose ou un pentose et R₂ est M, m vaut 1,

2. Esters selon la revendication 1, à la condition que, quand Z est un hexose ou un pentose, m vaut 0 et 1, en mélange avec des esters de l'acide sulfosuccinique ayant la formule : 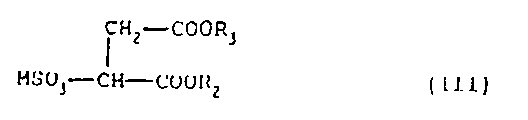 dans laquelle R₃ a les mêmes significations que R₁, sauf que p est égal à 0.

3. Esters de l'acide sulfosuccinique ayant la formule : 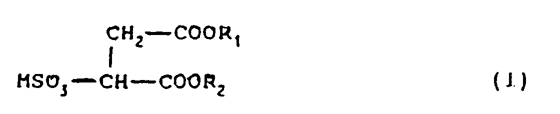 dans laquelle :
M est Na, K, Mg, NH₄ ou leurs mélanges ;
R₁ représente le groupe : 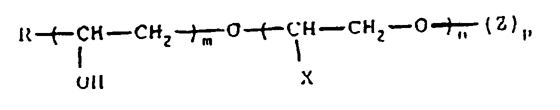
dans lequel :
R est un groupe alkyle ayant de 8 à 20 atomes de carbone,
X est H ou CH₃,
Z représente le résidu d'un hexose, d'un pentose ou du sorbitol,
m pouvant valoir 0 ou 1, n pouvant varier de 0 à 20 et p pouvant varier de 1 à 10,
R₂ est M ou R,
à la condition que, quand Z est le résidu du sorbitol, m = 1, n = 0 et p = 1 ; et à la condition que, quand Z est un hexose ou un pentose et R₂ est M, alors m vaut 1, ou n vaut de 1 à 20 et X est CH₃.

4. Compositions cosmétiques et/ou détergentes contenant un ou plusieurs esters de l'acide sulfosuccinique selon les revendications 1 à 3.

5. Compositions détergentes selon la revendication 4 dans lesquelles les esters de l'acide sulfosuccinique sont mélangés à d'autres agents tensioactifs.

## Revendications (Revendications pour l'(les) Etat(s) contractant(s) suivant(s): ES)

1. Procédé pour préparer des esters de l'acide sulfosuccinique ayant la formule : 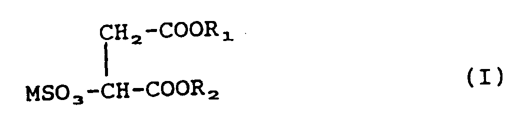 dans laquelle :
M est Na, K, Mg, NH₄ ou leurs mélanges ;
R₁ représente le groupe : 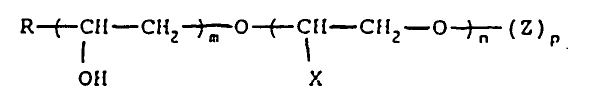
dans lequel :
R est un groupe alkyle ayant de 8 à 20 atomes de carbone,
X est H ou CH₃,
Z représente le résidu d'un hexose, d'un pentose ou du sorbitol,
m pouvant valoir 0 ou 1, n pouvant varier de 0 à 20 et p pouvant varier de 1 à 10,
R₂ est M ou R,
à la condition que, quand Z est le résidu du sorbitol, m = 1, n = 0 et p = 1, et que, quand Z est un hexose et un pentose et R₂ est M, m vaut 1,
qui comprend la sulfonation de différents diesters et semi-esters de l'acide maléique, ayant la formule : 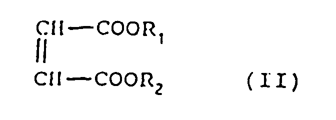 avec le sulfite ou le bisulfite requis du cation M, où R₁, R₂ et M ont les significations données ci-dessus.

2. Procédé pour préparer les esters selon la revendication 1, sauf que, quand Z est un hexose ou un pentose, m vaut 0 et 1, qui consiste en outre à mélanger les esters obtenus avec des esters de l'acide sulfosuccinique ayant la formule : 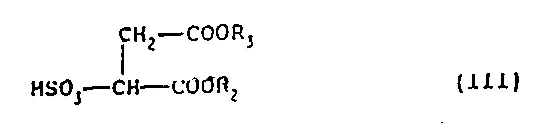 dans laquelle R₃ a les mêmes significations que R₁, sauf que p est égal à 0.

3. Procédé pour préparer les esters de l'acide sulfosuccinique ayant la formule : 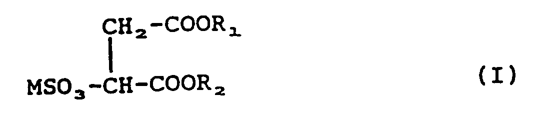 dans laquelle :
M est Na, K, Mg, NH₄ ou leurs mélanges ;
R₁ représente le groupe : 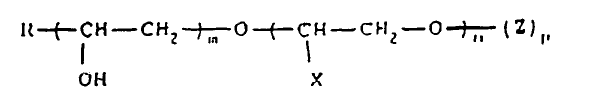
dans lequel :
R est un groupe alkyle ayant de 8 à 20 atomes de carbone,
X est H ou CH₃,
Z représente le résidu d'un hexose, d'un pentose ou du sorbitol,
m pouvant valoir 0 ou 1, n pouvant varier de 0 à 20 et p pouvant varier de 1 à 10,
R₂ est M ou R,
à la condition que, quand Z est le résidu du sorbitol, m = 1, n = 0 et p = 1 ; et à la condition que, quand Z est un hexose ou un pentose et R₂ est M, alors m vaut 1, ou n vaut de 1 à 20 et X est CH₃,
qui comprend la sulfonation de différents diesters et semi-esters de l'acide maléique, ayant la formule : 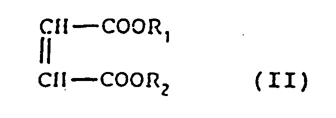 avec le sulfite ou le bisulfite requis du cation M, où R₁, R₂ et M ont les significations données ci-dessus.

4. Procédé pour préparer des compositions cosmétiques et/ou détergentes contenant un ou plusieurs esters de l'acide sulfosuccinique selon les revendications 1 à 3, qui consiste à mélanger au moins l'un desdits esters avec au moins un véhicule approprié.

5. Procédé pour préparer des compositions détergentes selon la revendication 4, dans lequel les esters de l'acide sulfosuccinique sont mélangés à d'autres agents tensioactifs.

## Revendications (Revendications pour l'(les) Etat(s) contractant(s) suivant(s): GR)

1. Procédé pour préparer des esters de l'acide sulfosuccinique ayant la formule : 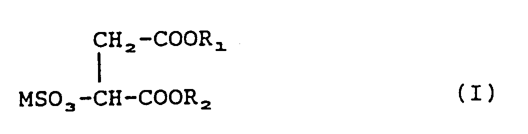 dans laquelle :
M est Na, K, Mg, NH₄ ou leurs mélanges ;
R₁ représente le groupe : 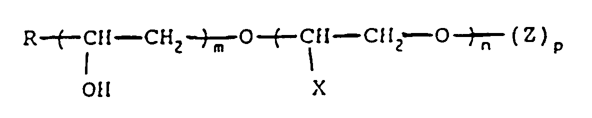
dans lequel :
R est un groupe alkyle ayant de 8 à 20 atomes de carbone,
X est H ou CH₃,
Z représente le résidu d'un hexose, d'un pentose ou du sorbitol,
m pouvant valoir 0 ou 1, n pouvant varier de 0 à 20 et p pouvant varier de 1 à 10,
R₂ est M ou R,
à la condition que, quand Z est le résidu du sorbitol, m = 1, n = 0 et p = 1,
qui comprend la sulfonation de différents diesters et semi-esters de l'acide maléique, ayant la formule : 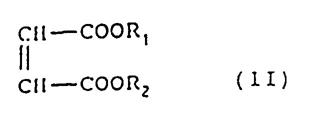 avec le sulfite ou le bisulfite requis du cation M, où R₁, R₂ et M ont les significations données ci-dessus.

2. Procédé pour préparer les esters selon la revendication 1, dans lequel Z représente le résidu du glucose, m et n valent chacun 0 et p est compris entre 1 et 5.

3. Procédé pour préparer les esters selon la revendication 1, qui consiste en outre à mélanger les esters obtenus avec des esters de l'acide sulfosuccinique ayant la formule : 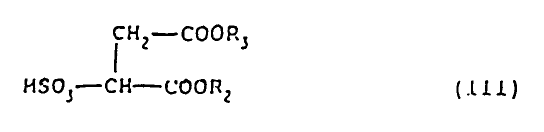 dans laquelle R₃ a les mêmes significations que R₁, sauf que p est égal à 0.

4. Procédé pour préparer les esters ayant la formule : 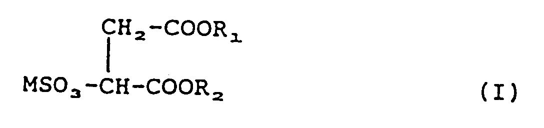 dans laquelle :
M est Na, K, Mg, NH₄ ou leurs mélanges ;
R₁ représente le groupe : 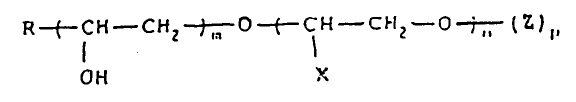
dans lequel :
R est un groupe alkyle ayant de 8 à 20 atomes de carbone,
X est H ou CH₃,
Z représente le résidu d'un hexose, d'un pentose ou du sorbitol,
m pouvant valoir 0 ou 1, n pouvant varier de 0 à 20 et p pouvant varier de 1 à 10,
R₂ est M ou R,
à la condition que, quand Z est le résidu du sorbitol, m = 1, n = 0 et p = 1 ; et à la condition que, quand Z est un hexose ou un pentose et R₂ est M, alors m vaut 1, ou n vaut de 1 à 20 et X est CH₃,
qui comprend la sulfonation de différents diesters et semi-esters de l'acide maléique, ayant la formule : 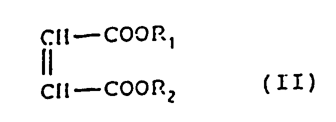 avec le sulfite ou le bisulfite requis du cation M, où R₁, R₂ et M ont les significations données ci-dessus.

5. Procédé pour préparer des compositions cosmétiques et/ou détergentes contenant un ou plusieurs esters de l'acide sulfosuccinique selon les revendications 1 à 4, qui consiste à mélanger au moins l'un desdits esters avec au moins un véhicule approprié.

6. Procédé pour préparer des compositions détergentes selon la revendication 5, dans lequel les esters de l'acide sulfosuccinique sont mélangés à d'autres agents tensioactifs.
